# EUROPÄISCHE PATENTSCHRIFT

(11) **EP 3 024 567 B1**
(45) Veröffentlichungstag und Bekanntmachung des Hinweises auf die Patenterteilung: **11.10.2017**
(21) Anmeldenummer: 14733527.7
(22) Anmeldetag: 27.06.2014
(51) Int. Cl.: B01J 13/14, B01J 13/20

(54) **VERKAPSELUNGSEINRICHTUNG UND -VERFAHREN ZUR VERKAPSELUNG EINER PROBE IN EINER POLYMERKAPSEL**
ENCAPSULATING DEVICE AND ENCAPSULATING METHOD FOR ENCAPSULATING A SAMPLE IN A POLYMER CAPSULE
DISPOSITIF ET PROCÉDÉ D'ENCAPSULATION D'UN ÉCHANTILLON DANS UNE CAPSULE POLYMÈRE

(30) Priorität: 26.07.2013 DE 102013012467
(43) Veröffentlichungstag der Anmeldung: 01.06.2016
(73) Patentinhaber: Fraunhofer-Gesellschaft zur Förderung der angewandten Forschung e.V., 80686 München (DE)
(72) Erfinder: ZIMMERMANN, Heiko, 97295 Waldbrunn (DE); NEUBAUER, Julia, 66386 St. Ingbert (DE)
(74) Vertreter: v. Bezold & Partner Patentanwälte - PartG mbB
(86) Internationale Anmeldenummer: PCT/EP2014/001776
(87) Internationale Veröffentlichungsnummer: WO 2015/010763

(56) Entgegenhaltungen:
- DE-A1- 10 203 629
- US-A1- 2005 282 264
- JENS M. KELM ET AL: "Method for generation of homogeneous multicellular tumor spheroids applicable to a wide variety of cell types", BIOTECHNOLOGY AND BIOENGINEERING, Bd. 83, Nr. 2, 20. Juli 2003 (2003-07-20), Seiten 173-180, XP055139337, ISSN: 0006-3592, DOI: 10.1002/bit.10655
- CHUAH A M ET AL: "Preparation of uniformly sized alginate microspheres using the novel combined methods of microchannel emulsification and external gelation", COLLOIDS AND SURFACES. A, PHYSICACHEMICAL AND ENGINEERING ASPECTS, ELSEVIER, AMSTERDAM, NL, Bd. 351, Nr. 1-3, 5. November 2009 (2009-11-05), Seiten 9-17, XP026705164, ISSN: 0927-7757, DOI: 10.1016/J.COLSURFA.2009.09.005 [gefunden am 2009-09-09]

## Beschreibung

Die Erfindung betrifft eine Verkapselungseinrichtung, die zur Verkapselung einer Probe, insbesondere umfassend mindestens eine biologische Zelle, in einer Polymerkapsel, zum Beispiel in einer Alginat-Kapsel, eingerichtet ist. Des Weiteren betrifft die Erfindung ein Verfahren zur Verkapselung einer Probe in einer Polymerkapsel, insbesondere unter Verwendung der genannten Verkapselungseinrichtung. Anwendungen der Erfindung sind insbesondere bei der Verkapselung von biologisch wirksamen Proben, z. B. von biologischen Zellen, wie zum Beispiel pluripotenten Stammzellen oder Primärgewebe-Zellen, in biokompatiblen Polymeren, wie zum Beispiel Alginat gegeben.

Es ist allgemein bekannt, biologisch wirksame Substanzen, wie zum Beispiel Enzyme, Proteine, biologische Zellen, Zellbestandteile oder Zellgruppen für Anwendungen bei chemischen, biochemischen oder medizinischen Verfahren zu immobilisieren. Die Immobilisierung kann eine vollständige Umhüllung (Verkapselung) der Substanz in einem Matrixmaterial (Verkapselungssubstanz) umfassen. Beispielsweise ist bekannt, für Anwendungen in der regenerativen Medizin, Zellen, Gewebe oder Mikroorgane als Transplantate in einer biokompatiblen Verkapselungssubstanz einzubetten. Die Verkapselung bietet vorteilhafterweise die Möglichkeit, die Transplantate vor einer Immunantwort eines Empfängers und vor mechanischem Stress zu schützen, während gleichzeitig die Nährstoffversorgung des Transplantats gewährleistet bleibt.

Häufig ist eine Verkapselung in dreidimensionalen, zum Beispiel kugelförmigen, Kapseln von Interesse, da die dreidimensionale Umgebung für biologische Zellen einer physiologischen Umgebung ähnelt, so dass sich die Zellen besser erhalten als in einer schichtförmigen Verkapselung, zum Beispiel auf der Oberfläche eines Substrats. Beispielsweise ist bekannt, das Chondrozyten nur in einer dreidimensionalen Umgebung das für den Aufbau von Knorpel typische Kollagen produzieren, was einen Grund für die verzögerte Heilung von Knorpeldefekten ist.

Als Verkapselungssubstanz wird häufig das Polymer Alginat verwendet. Alginat ist neben Zellulose ein strukturgebender Hauptbestandteil der Zellwände mariner Braunalgen (Phaeophyceae). Alginat ist ein saures Polysaccharid, dass sich aus 1-4-verknüpften α-L-Guluronsäure- und β-D-Mannuronsäure-Ketten zusammensetzt. Es werden Homopolymere (MM- oder GG-Blöcke) und heteropolymere Bereiche (MG-Blöcke) gebildet. Die Carboxylgruppen der Säureketten können durch bivalente Kationen (zum Beispiel Ca²⁺, Ba²⁺, Fe²⁺) vernetzt werden. Im vernetzen Zustand bildet Alginat ein Hydrogel, dass sich als vorteilhaft für die Verkapselung biologisch wirksamer Substanzen erwiesen hat. Die Viskosität des vernetzten Alginats steigt mit zunehmender Konzentration der Kationen an, wobei bei physiologischen Temperaturen mit Kalzium oder Barium vernetztes Alginat stabil ist.

Es sind verschiedene Verfahren bekannt, zum Beispiel biologische Zellen in Alginat zu verkapseln. Beispielsweise ist bekannt, Tropfen einer Suspension biologischer Zellen in einer Alginatlösung mittels Druckluft zu erzeugen und in ein Bad einer Polymerisationssubstanz mit bivalenten Kationen zu bewegen, wo es zu einer Vernetzung der Alginatketten kommt (siehe U. Zimmermann et al. in "Ann. N.Y. Acad. Sci.", Band 944, 2001, S. 199-215). Bei einem weiteren bekannten Verfahren werden fallende Tropfen einer Zell-Alginat-Suspension mit Kristalliten, die bivalente Kationen enthalten, beschossen, wodurch eine innere Vernetzung der Tropfen gefördert wird (siehe H. Zimmermann et al. in "Biomaterials" Bd. 28, 2007, S. 1327-1345).

Die herkömmlichen Verfahren zur Verkapselung von Zellen haben mehrere Nachteile. Erstens zeichnen sich die herkömmlichen Techniken durch einen hohen Verbrauch von Zellen und von Alginatlösung aus. Bei der Erzeugung der fallenden Suspensionstropfen ist nicht in jedem Tropfen die gewünschte Zelle oder die gewünschte Anzahl von Zellen enthalten. Die Reproduzierbarkeit der Verkapselung ist von zahlreichen Verfahrensbedingungen und insbesondere den Fertigkeiten der Person abhängig, welche die Verkapselung durchführt. Schließlich bestehen Beschränkungen bei der Verbindung der herkömmlichen Verkapselungsverfahren mit komplexen Prozessen bei der Kultivierung (Vermehrung, Wachstum und/oder Differenzierung) biologischer Zellen. Letzteres ist insbesondere bei der Handhabung von therapeutisch relevanten Zellen, wie zum Beispiel von Stammzellen, von Bedeutung. Insbesondere für die Verkapselung von Stammzellen besteht ein Interesse an einer dreidimensionalen Einbettung, da diese Vorteile für die Proliferation und Entwicklung der Zellen erwarten lässt.

Eine weitere bekannte Anwendung der Verkapselung in biokompatiblen Polymeren, zum Beispiel Alginat, ist bei der Kryokonservierung gegeben. Durch die schützende Umhüllung der Zellen kann mechanischer Stress durch eine eventuelle extrazellulare Eiskristallbildung reduziert werden, und es können bei der Kryokonservierung von Zellaggregaten Zell-Zell-Kontakte geschützt werden.

Herkömmliche Immobilisierungstechniken sind nicht auf die Verkapselung biologisch wirksamer Substanzen in Alginat beschränkt. Auch bei anderen Aufgaben der Verkapselung von Substanzen in Polymeren, wie zum Beispiel von Insulin in Chitosan, besteht ein Interesse an einer Einbettung in dreidimensionalen, zum Beispiel kugel- oder tropfenförmigen, Kapseln. Es ist des Weiteren bekannt, Zellen in hängenden Tropfen zu kultivieren, z. B. für die definierte Bildung und Kultivierung von dreidimensionalen, multizellulären Aggregaten. Die herkömmlichen Techniken sind bisher ausschließlich auf die Kultivierung der Zellen in den hängenden Tropfen beschränkt. DE 102 03 629 A1 offenbart eine Vorrichtung zur Herstellung von Mikrokapseln, umfassend eine erste Abgabeeinrichtung umfassend eine Verkapselungsdüse und eine Vernetzungseinrichtung. DE 102 03 629 A1 offenbart auch ein Verfahren zur Herstellung von Mikrokapseln aus vernetzten Alginaten, umfassend die Schritte 1) Bereitstellung eines Tropfens aus Alginat-Zell-Gemisch; und 2) Vernetzung des Tropfens durch Zufuhr eines Vernetzungsmittels zu dem Tropfen. Die Aufgabe der Erfindung ist es, eine verbesserte Verkapselungseinrichtung bereitzustellen, mit der Nachteile herkömmlicher Techniken vermieden werden. Die Verkapselungseinrichtung soll insbesondere eine Verkapselung mit verbesserter Ausbeute, erhöhter Reproduzierbarkeit und/oder verminderter Belastung für die verkapselte Probe ermöglichen. Des Weiteren besteht speziell ein Interesse an einer verbesserten Verkapselungseinrichtung, die für die Verkapselung von Proben, umfassend mindestens eine biologische Zelle, geeignet ist und eine Integration in Prozesse zur Handhabung biologischer Zellen vereinfacht. Eine weitere Aufgabe der Erfindung ist es, ein verbessertes Verfahren zur Verkapselung einer Probe in einer Polymerkapsel bereitzustellen, mit dem Nachteile herkömmlicher Verfahren vermieden werden. Das Verkapselungsverfahren soll sich insbesondere durch eine erhöhte Ausbeute, verbesserte Reproduzierbarkeit und/oder verminderte Belastung der verkapselten Proben auszeichnen.

Diese Aufgaben werden durch eine Verkapselungseinrichtung und/oder ein Verkapselungsverfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die genannte Aufgabe durch eine Verkapselungseinrichtung gelöst, die zur Verkapselung einer Probe in einer Polymerkapsel eingerichtet ist und einen Tropfengenerator und eine Vernetzungseinrichtung umfasst. Der Tropfengenerator ist für die Bildung eines Tropfens einer Suspension eingerichtet, der die zu verkapselnde Probe enthält. Gemäß der Erfindung weist der Tropfengenerator eine Halteeinrichtung auf, die für eine Aufnahme des Tropfens in einem hängenden Zustand eingerichtet ist. Die Vernetzungseinrichtung ist für eine Polymerisierung des Tropfens eingerichtet. Gemäß der Erfindung ist die Vernetzungseinrichtung angeordnet, eine Polymerisationssubstanz zu dem hängenden Tropfen an der Halteeinrichtung zuzuführen und die Polymerkapsel (polymerisierter Tropfen) zu bilden.

Gemäß einem zweiten Gesichtspunkt der Erfindung wird die genannte Aufgabe durch ein Verfahren zur Verkapselung einer Probe in einer Polymerkapsel gelöst, bei dem ein Tropfen einer Suspension, der die zu verkapselnde Probe enthält, in einem hängenden Zustand bereitgestellt und als hängender Tropfen mit einer Polymerisationssubstanz polymerisiert wird, so dass die Polymerkapsel gebildet wird. Vorzugsweise wird das erfindungsgemäße Verfahren mit der Verkapselungseinrichtung gemäß dem obigen ersten Gesichtspunkt der Erfindung ausgeführt.

Die Polymerisierung des Suspensionstropfens erfolgt, wenn dieser an der Halteeinrichtung im hängenden Zustand positioniert ist. Die Halteeinrichtung bildet allgemein eine Aufnahme für den hängenden Suspensionstropfen. Der hängende Zustand des Suspensionstropfens bedeutet, dass eine Oberseite des Suspensionstropfens, das heißt eine in Bezug auf die Vertikalrichtung nach oben (entgegengesetzt zur Gravitation) weisende Seite, an der Halteeinrichtung haftet. Der hängende Tropfen ist an der Halteeinrichtung unbewegt. Die Unterseite des hängenden Tropfens, das heißt die in Bezug auf die Vertikalrichtung nach unten (in Richtung der Gravitation) weisende Seite, kann vollständig frei liegen (frei hängender Tropfen) oder durch ein zusätzliches Halteelement unterstützt sein (unterstützter hängender Tropfen), wobei die Form des hängenden Tropfen in jedem Fall durch die Oberflächenspannung der Suspension in Zusammenwirkung mit der Halteeinrichtung und gegebenenfalls dem Halteelement bestimmt wird.

Die Bereitstellung des hängenden Tropfens erlaubt, dass im Unterschied zu den herkömmlichen Techniken der Suspensionstropfen in einem ruhenden Zustand bereitgestellt wird. Der Tropfen hat eine feste Position relativ zur Vernetzungseinrichtung. Dies ermöglicht, vor der Polymerisierung sicherzustellen, dass Tropfen die zu verkapselnde Probe enthält. Die Polymerisierung von Tropfen, die etwa keine Probe enthalten, wird ausgeschlossen. Die Tropfenform wird reproduzierbar durch Eigenschaften der Suspension und der Halteeinrichtung (gegebenenfalls mit dem Halteelement) bestimmt. Dies erlaubt, die Bedingungen der Zufuhr der Polymerisationssubstanz zu dem hängenden Tropfen mit erhöhter Reproduzierbarkeit einzustellen, was sich vorteilhaft auf die Reproduzierbarkeit der Verkapselung auswirkt. Des Weiteren werden unerwünschte Scherkräfte, wie sie bei der herkömmlichen Bildung von Suspensionstropfen, zum Beispiel mit Druckluft, auftreten können, durch die schonende Platzierung der Probe im hängenden Tropfen vermieden.

Ein weiterer Vorteil, der sich insbesondere bei der Verkapselung biologischer Proben auswirkt, besteht darin, dass die Polymerisierung mit der Kultivierung (Vermehrung, Wachstum und/oder Differenzierung) biologischer Zellen, insbesondere mit der Kultivierung im hängenden Tropfen kombinierbar ist.

Die Verkapselungseinrichtung kann als Teil einer Kultivierungseinrichtung für biologische Zellen konfiguriert sein, in der die Zellen in hängenden Tropfen kultiviert und gegebenenfalls verkapselt werden.

Vorteilhafterweise kann die Verkapselungseinrichtung zur Verkapselung einer einzelnen Probe in einem einzelnen Tropfen eingerichtet sein. In diesem Fall bildet die Halteeinrichtung eine Aufnahme für einen einzigen Tropfen. Alternativ kann die Verkapselungseinrichtung für die gleichzeitige oder aufeinanderfolgende Verkapselung einer Vielzahl von Proben in einer Vielzahl von Tropfen eingerichtet sein. In diesem Fall bildet die Halteeinrichtung eine Aufnahme für mehrere Tropfen im hängenden Zustand. Wenn bei der Beschreibung der vorliegenden Erfindung auf die Verkapselung einer Probe in einem Tropfen Bezug genommen wird, soll damit die Verkapselung einer Vielzahl von Proben in einer Vielzahl von Tropfen in entsprechender Weise umfasst sein.

Vorteilhafterweise bestehen verschiedene Möglichkeiten, die Halteeinrichtung zur Aufnahme des hängenden Tropfens zu gestalten. Gemäß einer ersten Variante umfasst die Halteeinrichtung eine Halteplatte mit einer freiliegenden Unterseite, die für die Aufnahme des hängenden Tropfens eingerichtet ist. Die Halteplatte, die zum Beispiel aus transparentem Material, wie zum Beispiel aus Glas oder Kunststoff hergestellt sein kann, hat den Vorteil, dass der hängende Tropfen an seiner Oberseite gegen unerwünschte Einflüsse geschützt ist. Die freiliegende Unterseite der Halteplatte kann zur Lokalisierung des hängenden Tropfens an einer bestimmten Position mit einer hydrophilen Oberfläche ausgestattet sein.

Gemäß einer zweiten Variante der Erfindung kann die Halteeinrichtung eine Lochplatte aufweisen, die ein Loch für die Aufnahme des hängenden Tropfens oder - für die Verkapselung einer Vielzahl von Proben - eine Vielzahl von Löchern jeweils für die Aufnahme eines hängenden Tropfens aufweist. Der Suspensionstropfen wird unter der Wirkung der Oberflächenspannung in dem Loch gehalten. Die Verwendung der Lochplatte hat den Vorteil, dass beidseitig der Lochplatte ein Zugriff auf den hängenden Tropfen, beispielsweise für die Zufuhr von Substanzen und/oder für eine Zufuhr der Probe, gegeben ist.

Gemäß einer dritten Variante der Erfindung kann die Halteeinrichtung eine Netzplatte (Gitterplatte) umfassen, die für die Aufnahme des hängenden Tropfens eingerichtet ist. Die Netzplatte wird durch eine Netzstruktur mit einer Vielzahl von Öffnungen gebildet, wobei jeweils eine Gruppe von Öffnungen eine Aufnahme für den hängenden Tropfen bildet. Da der Tropfen im hängenden Zustand Kontakt mit mehrere Netzstäben hat, kann sich die Netzplatte durch eine zum Beispiel im Vergleich zur Halteplatte vergrößerte Haltekraft auszeichnen.

Schließlich kann die Halteeinrichtung gemäß einer weiteren Variante eindimensional in Gestalt eines Haltestabes gebildet sein, der für die Aufnahme des hängenden Tropfens, vorzugsweise für die Aufnahme einer Reihe hängender Tropfen eingerichtet ist.

Die genannten Varianten können einzeln oder in Kombinationen bereitgestellt werden. Beispielsweise kann die Halteeinrichtung in einem ersten Abschnitt eine geschlossene Halteplatte für hängende Tropfen und mit einem zweiten Abschnitt eine nicht-geschlossene Platte, wie zum Beispiel eine Lochplatte oder eine Netzplatte aufweisen. Des Weiteren kann die Halteeinrichtung mit dem zusätzlichen, stützenden Halteelement auf der Unterseite zum Beispiel der Halteplatte, der Lochplatte, der Netzplatte oder des Haltestabs ausgestattet sein.

Gemäß einer bevorzugten Ausführungsform der Erfindung weist der Tropfengenerator eine Zufuhreinrichtung auf, mit der die Suspension zu der Halteeinrichtung geleitet werden kann. Die Zufuhreinrichtung ist für die Bildung des hängenden Tropfens an der Halteeinrichtung eingerichtet. Vorteilhafterweise wird der Betrieb des Tropfengenerators durch die Zufuhreinrichtung vereinfacht. Besonders bevorzugt ist eine Ausführungsform der Erfindung, bei der die Zufuhreinrichtung eine Pipettiereinrichtung (erste Pipettiereinrichtung) umfasst, die für ein Setzen des hängenden Tropfens an der Halteeinrichtung angeordnet ist. Vorteilhafterweise wird durch die Verwendung der ersten Pipettiereinrichtung die Verbindung der Verkapselung mit weiteren Prozessschritten der Handhabung biologischer Zellen vereinfacht. Im Gegensatz zu den herkömmlichen Techniken kann auf die Verwendung zusätzlicher Geräte, wie zum Beispiel eines Druckluftgenerators zur Tropfenerzeugung, verzichtet werden.

Die Vernetzungseinrichtung der erfindungsgemäßen Verkapselungseinrichtung ist allgemein dafür ausgelegt, die Polymerisationssubstanz mit der Suspension im hängenden Zustand in Kontakt zu bringen. Vorteilhafterweise bestehen verschiedene Möglichkeiten, die Polymerisationssubstanz dem hängenden Tropfen zuzuführen. Gemäß einer ersten Variante umfasst die Vernetzungseinrichtung eine Pipettiereinrichtung (zweite Pipettiereinrichtung), mit der die Polymerisationssubstanz im flüssigen Zustand in den hängenden Tropfen eingeleitet werden kann. Beispielsweise kann die Halteeinrichtung mit der oben genannten ersten Pipettiereinrichtung zum Setzen des hängenden Tropfens und der zweiten Pitpettiereinrichtung zur Zufuhr der Polymerisationssubstanz in den hängenden Tropfen ausgestattet sein. Die Verwendung der zweiten Pipettiereinrichtung kann Vorteile in Bezug auf eine schonende Zufuhr der Polymerisationssubstanz und/oder die Zufuhr eines definierten Volumens der Polymerisationssubstanz haben.

Gemäß einer zweiten Variante kann die Vernetzungseinrichtung eine Partikelschusseinrichtung aufweisen, die für ein Einschießen der Polymerisationssubstanz in Gestalt fester Partikel (Kristallite) in den hängende Tropfen eingerichtet ist. In diesem Fall können sich Vorteile für eine räumliche Trennung der Vernetzungseinrichtung vom hängenden Tropfen und die Vermeidung einer unbeabsichtigten Kontamination des hängenden Tropfens mit Fremdsubstanzen ergeben.

Gemäß einer weiteren Variante kann die Polymerisationssubstanz in einer Wanne bereitgestellt werden. Die Wanne ist unterhalb der Halteeinrichtung positioniert, und die Halteeinrichtung und die Wanne sind relativ zueinander beweglich, so dass der hängende Tropfen in die Polymerisationssubstanz in der Wanne eingetaucht werden kann. Diese Gestaltung der Vernetzungseinrichtung kann Vorteile für die gleichzeitige Polymerisierung einer Vielzahl von Tropfen, das heißt für eine gleichzeitige Verkapselung einer Vielzahl von Proben, haben.

Die Erfinder haben festgestellt, dass der Tropfen auch nach der Polymerisierung, d.h. die Polymerkapsel, an der Halteeinrichtung haften kann. Die Anhaftung der Polymerkapsel wird insbesondere bei der Verwendung einer Lochplatte oder einer Netzplatte als Halteeinrichtung begünstigt. Vorteilhafterweise kann die Polymerkapsel an der Halteeinrichtung weiteren Bearbeitungsschritten, insbesondere einer Umhüllung mit einer flüssigen Hüllsubstanz, unterzogen werden. Hierzu ist die Halteeinrichtung gemäß einer weiteren bevorzugten Ausführungsform der erfindungsgemäßen Verkapselungseinrichtung zusätzlich mit einer Hülleinrichtung ausgestattet, die für die Umhüllung der Polymerkapsel mit der Hüllsubstanz eingerichtet ist.

Vorzugsweise umfasst die Hülleinrichtung mindestens eine Fluidleitung zur Zufuhr der Hüllsubstanz zur Polymerkapsel, wobei die Hüllleitung mit der Halteeinrichtung fest verbunden ist. Alternativ kann eine dritte Pipettiereinrichtung zur Zufuhr der Hüllsubstanz vorgesehen sein.

Gemäß einer weiteren vorteilhaften Ausführungsform der Erfindung ist die Verkapselungseinrichtung mit einer Messeinrichtung ausgestattet. Die Messeinrichtung, basierend zum Beispiel auf einer optischen oder elektrischen Messung, ist für eine Untersuchung des hängenden Tropfens und/oder der Polymerkapsel vorgesehen. Vorteilhafterweise ermöglicht die Messeinrichtung eine Überprüfung, ob sich vor der Polymerisierung des Tropfens in diesem die gewünschte Probe befindet, der hängende Tropfen die gewünschte Gestalt hat und/oder die Polymerisierung zu einer ausreichenden Verkapselung der Probe geführt hat.

Weitere Vorteile für den Gebrauch der Verkapselungseinrichtung ergeben sich, wenn diese gemäß einer weiteren Ausführungsform der Erfindung mit einer Trenneinrichtung ausgestattet ist, mit der die Polymerkapsel von der Halteeinrichtung abtrennbar ist. Die Trenneinrichtung, die zum Beispiel einen Schaber auf der Unterseite der Halteeinrichtung umfasst, erlaubt die Überführung der Polymerkapsel in ein Sammelgefäß zur Vorbereitung der weiteren Verwendung, zum Beispiel für Transplantationszwecke oder für die Kryokonservierung der verkapselten Probe.

Die Erfindung kann mit einer Vielzahl von Polymeren zur Verkapselung von Proben realisiert werden. Die Probe umfasst allgemein ein synthetisches und/oder natürliches Material. Vorzugsweise umfasst die Probe biologisch wirksames Material, insbesondere mindestens eine biologische Zelle, Zellbestandteile, in Zellen wirksame Makromoleküle, wie zum Beispiel Enzyme, Proteine oder Nukleinsäuren, Zellgruppen, Zellgewebe oder aus Zellgruppen zusammengesetzte Organe. Als Verkapselungssubstanz können verfügbare Polymere, insbesondere biokompatible Polymere, wie zum Beispiel Alginat, Gelatine, Agarose, Chitosan, oder Silikon verwendet werden.

Die Suspension, in der die Probe enthalten ist, kann eine Polymerlösung, insbesondere eine Alginatlösung, umfassen, während die Polymerisationssubstanz ein Vernetzungsmittel, insbesondere vernetzende Ionen zur Vernetzung von Alginat, enthält. In diesem Fall wird der hängende Tropfen durch die Polymerlösung, insbesondere Alginatlösung, gebildet, in dem die Probe suspendiert ist. Alternativ kann die Suspension das Vernetzungsmittel, insbesondere vernetzende Ionen zur Vernetzung von Alginat, enthalten, während die Polymerisationssubstanz eine Alginatlösung umfasst, die nach Zufuhr zum hängenden Tropfen polymerisiert wird.

Die erfindungsgemäße Verkapselung von Proben, insbesondere von biologischen Zellen, hat allgemein die folgenden Vorteile. Die Verkapselung von Zellen kann definiert und reproduzierbar durchgeführt werden, indem die Zahl der Zellen oder Zellaggregate pro hängenden Tropfen kontrolliert wird. Die Sedimentation der Probe im hängenden Tropfen kann für eine gezielte Positionierung der Probe im verkapselten Zustand ausgenutzt werden. Alternativ kann durch ein Resuspendieren der Zellsuspension unmittelbar vor der Vernetzung eine gleichmäßige Verteilung der Probe, insbesondere der Zellen oder Zellaggregate, im hängenden Tropfen gewährleistet werden. Somit ist bei Bedarf auch - durch die Integration geeigneter Pipettierschritte - eine Lage der Probe in der äußersten Peripherie vermeidbar.

Bei der Verkapselung kommt es zu einer exakten Positionierung und Trennung von hängenden Tropfen. Nach der Vernetzung bleiben die Polymerkapsel vorzugsweise an der Plattenunterseite haften, so dass sie für eine weitere Verwendung gezielt und spezifisch abgetrennt werden können. Im Gegensatz zu konventionellen Verfahren, bei der man eine unbekannte Anzahl an Alginat-Kapseln erhält, die anschließend auf Grund ihrer geringen Größe kaum separiert werden können, ermöglicht die erfindungsgemäße Verkapselung eine exakte Anzahl an Poymerkapseln mit einer definierten Größe, die sich z. B. durch das zuvor pipettierte Tropfenvolumen ergibt.

Die Bildung von Kapseln ohne Proben wird ausgeschlossen, und eine nachträgliche Überprüfung oder Trennung nicht beladener Kapsel ist nicht erforderlich. Die erfindungsgemäß hergestellten Polymerkapseln haben eine definierte Größe. Bei Verkapselung einer Vielzahl von Proben haben die Kapseln eine nahezu gleiche Größe. Schließlich ermöglicht die Erfindung die Verkapselung von Zellen während oder nach der Kultivierung im hängenden Tropfen, wobei etwa eine enzymatische Ablösung von Wachstumsoberflächen nicht erforderlich ist. Belastungen der Zellen werden vermieden.

Ein wichtiger Vorteil der Verkapselung in hängenden Tropfen ist ferner, dass sie einfach in bestehende Zellkulturabläufe integriert werden kann, da der hängende Tropfen ursprünglich für die Kultivierung und Differenzierung von Zellen vorgeschlagen und entwickelt wurde. Während diese Methode vor der Erfindung auf Anwendungen begrenzt war, die keine Adhäsion von Zellen auf Oberflächen benötigen, da innerhalb des Tropfens nur die Bildung von dreidimensionalen Zellaggregaten möglich ist, werden durch die Integration von Microcarriern - meist sphärische Trägerpartikel mit einem Durchmesser im bereich von 50 µm bis 500 µm, auf deren Oberfläche sich die Zellen anheften können - sind nun nahezu alle Zellkulturprozesse in den hängenden Tropfen übertragbar. Beispielsweise können Zellen zunächst entweder in Suspension oder zusammen mit Microcarriern im hängenden Tropfen kultiviert werden. Dazu werden die Tropfen der entsprechenden Zellsuspension entweder manuell oder mit Hilfe eines Pipettierroboters z. B. an die Tropfenplatte der Halteeinrichtung gesetzt. Anschließend kann eine automatische oder manuelle Kultivierung, Differenzierung oder ein Hochdurchsatz-Screening nach Wirkstoffen im Rahmen von Medikamentenentwicklungen durchgeführt werden. Das dabei entstehende zelluläre Endprodukt oder auch jegliche Intermediärprodukte können jederzeit in Alginat verkapselt werden, indem das Kultur- oder Differenzierungsmedium durch die Alginatlösung ersetzt und gemäß der Erfindung vernetzt wird.

Weitere Einzelheiten und Vorteile der Erfindung werden im Folgenden unter Bezug auf die beigefügten Zeichnungen beschrieben. Es zeigen:
- Figur 1:: die Bildung eines hängenden Tropfens gemäß einer Ausführungsform der Erfindung;
- Figur 2:: eine erste Variante der Polymerisierung des hängenden Tropfens gemäß Figur 1;
- Figur 3:: eine weitere Variante der Polymerisierung des hängenden Tropfens gemäß Figur 1;
- Figur 4:: die Umhüllung einer Polymerkapsel mit einer Hüllsubstanz;
- Figur 5:: eine Kultivierung von Zellen in einer Polymerkapsel mit einem Kultivierungsmedium;
- Figuren 6 und 7:: Varianten optischer oder elektrischer Messungen am hängenden Tropfen; und
- Figuren 8 bis 10:: Varianten der Abtrennung des polymerisierten Tropfens von der Halteeinrichtung.

Ausführungsformen der Erfindung werden im Folgenden insbesondere unter Bezug auf die Gestaltung der Verkapselungseinrichtung und die Polymerisierung von Tropfen im frei hängenden Zustand beschrieben. Dabei wird beispielhaft auf die Verkapselung biologischer Zellen in Alginatkapseln Bezug genommen. Es wird betont, dass die Erfindung nicht auf diese Anwendung beschränkt ist, sondern entsprechend mit anderen Proben, insbesondere biologisch wirksamen Substanzen und/oder anderen Materialien, und/oder anderen Polymeren und/oder mit unterstützt hängenden Tropfen möglich ist. Einzelheiten der Auswahl des Polymermaterials und des Vernetzungsmittels, insbesondere die Einstellung von Konzentrationen oder die Auswahl von bivalenten Kationen, und Einzelheiten der Handhabung von biologischen Zellen werden nicht beschrieben, da diese an sich von herkömmlichen Techniken bekannt sind.

Die Erfindung wird insbesondere in Bezug auf den Tropfengenerator mit der Halteeinrichtung und die Vernetzungseinrichtung und deren gegenseitiges Zusammenwirken beschrieben. Die Verkapselungseinrichtung kann weitere Komponenten, wie zum Beispiel Flüssigkeitsreservoire, Pumpen, Temperierungseinrichtungen, Befeuchtungseinrichtungen und dergleichen aufweisen. Die Verkapselungseinrichtung kann insbesondere ein autarkes Gerät für Labor- oder Industrieanwendungen zur Herstellung von verkapselten Proben in Polymerkapseln umfassen. Alternativ kann die Verkapselungseinrichtung Teil eines Kultivierungsgerätes für biologische Zellen sein, das insbesondere für die Kultivierung von Zellen in hängenden Tropfen ausgelegt ist. In diesem Fall bildet der Tropfengenerator mit der Halteeinrichtung zugleich ein Substrat für die Kultivierung von Zellen in hängenden Tropfen.

Die Ausführungsformen der Erfindung werden im Folgenden unter beispielhaftem Bezug auf die Bereitstellung und Polymerisierung einzelner hängender Tropfen beschrieben. Es wird betont, dass die Erfindung entsprechend mit Gruppen hängender Tropfen ausführbar ist. Für die massenhafte Herstellung von Proben in Polymerkapseln können zahlreiche, zum Beispiel 100, 1000 oder mehr, hängende Tropfen gleichzeitig bereitgestellt und zeitlich parallel oder aufeinanderfolgend polymerisiert werden.

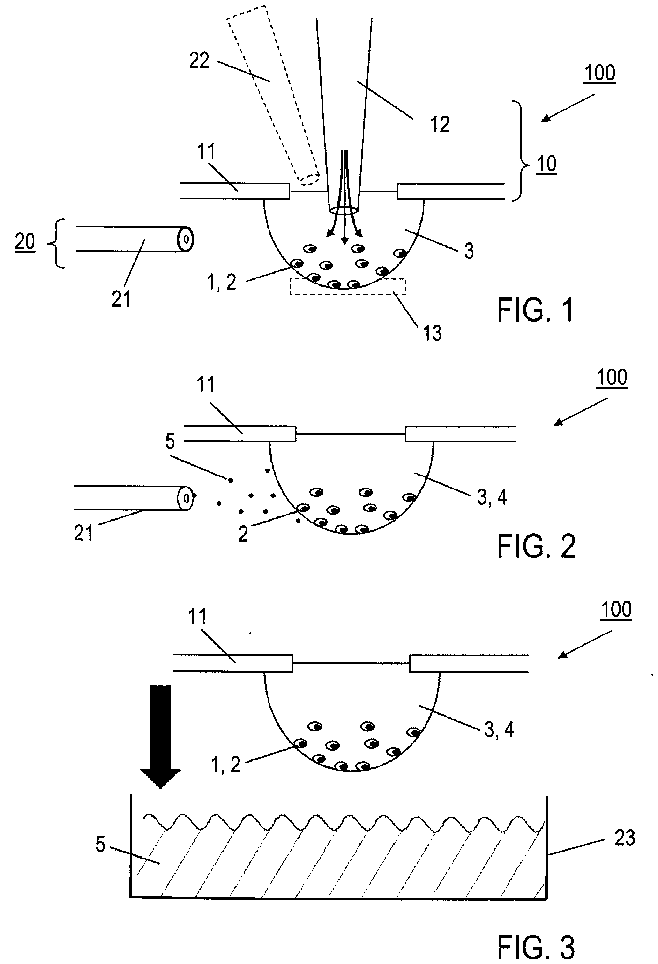

Figur 1 illustriert Merkmale bevorzugter Ausführungsformen der erfindungsgemäßen Verkapselungseinrichtung 100 (teilweise gezeigt) mit einem Tropfengenerator 10 und einer Vernetzungseinrichtung 20. Der Tropfengenerator 10 umfasst eine Halteeinrichtung 11 in Gestalt einer Lochplatte. Des Weiteren ist die Halteeinrichtung 11 bei der dargestellten Ausführungsform mit einer Zufuhreinrichtung 12, umfassend eine erste Pipettiereinrichtung, ausgestattet. Die Zufuhreinrichtung 12 kann in Bezug auf die Halteeinrichtung 11 beweglich oder mit dieser fest verbunden sein. Die Lochplatte ist eine ebene, feste Platte mit mindestens einem Loch, die sich in der Verkapselungseinrichtung 100 in horizontaler Richtung erstreckt. Die Dicke der Platte beträgt zum Beispiel 3 mm, und der Durchmesser des mindestens einen Loches beträgt zum Beispiel 100 µm bis 5 mm. Die Lochplatte ist zum Beispiel eine Tropfenplatte, die kommerziell für die Kultivierung hängender Tropfen verfügbar ist.

In Figur 1 ist ein optional vorgesehenes Halteelement 13 (gestrichelt gezeigt) illustriert. Das Halteelement 13 kann in Gestalt z. B. einer Platte oder eines Stabes zur Unterstützung des Tropfens (unterstützter hängender Tropfen) vorgesehen sein.

Die Vernetzungseinrichtung 20 umfasst zum Beispiel eine Partikelschusseinrichtung 21 und/oder eine zweite Pipettiereinrichtung 22 (gestrichelt gezeigt). Die Wahl des Typs der Vernetzungseinrichtung 20 erfolgt in Abhängigkeit von der konkreten Anwendung der Erfindung. Beispielsweise wird ein Einschießen von festen Partikeln der Polymerisationssubstanz mit der Partikelschusseinrichtung 21 bevorzugt, wenn eine schnelle Polymerisierung des gesamten Volumens des hängenden Tropfens gewünscht wird. Die Verwendung der zweiten Pipettiereinrichtung 22 hingegen kann Vorteile haben, wenn eine langsame Polymerisierung von einer Seite des hängenden Tropfens her bevorzugt wird. Beide Typen von Vernetzungseinrichtungen können kombiniert werden. Des Weiteren kann eine Vernetzung in einer Wanne vorgesehen sein (siehe unten, Figur 3).

Für die Verkapselung einer Probe, umfassend biologische Zellen 2 (Beispiele siehe unten) wird zunächst mit der Zufuhreinrichtung 12 an der Lochplatte der Halteeinrichtung 11 ein hängender Tropfen 3 gesetzt. Die Zufuhreinrichtung 12 kann manuell oder mit einer von einem Prozessor gesteuerten Antriebseinrichtung (Pipettierroboter) betätigt werden. Es wird zum Beispiel ein Tropfen mit einem Durchmesser von 4 mm mit 1000 suspendierten Zellen gebildet. Die Zufuhreinrichtung 12 kann des Weiteren verwendet werden, um dem Tropfen 3 weitere Substanzen, wie zum Beispiel Nährstoffe oder synthetische Partikel (so genannte "Microcarrier") zuzusetzen.

Nachdem der Tropfen 3 im hängenden Zustand gebildet ist, erfolgt die Polymerisierung der Alginatlösung durch die Zufuhr einer Polymerisationssubstanz 5 (Figur 2). Die Polymerisationssubstanz 5 umfasst Kristallite, welche bivalente Kationen enthalten. Die Partikelschusseinrichtung 21 enthält zum Beispiel ein Kristallitreservoir (nicht dargestellt) aus dem mit Hilfe einer Druckluftquelle (nicht dargestellt) Kristallite durch ein Lenkrohr in den hängenden Tropfen 3 geschossen werden.

Figur 3 zeigt eine abgewandelte Ausführungsform der Erfindung, bei der die Vernetzungseinrichtung 20 eine Wanne 23 umfasst, in der die Polymerisationssubstanz 5 als flüssiges Vernetzungsmittel angeordnet ist. In diesem Fall erfolgt die Vernetzung des hängenden Tropfens 3 mit der Probe 1 in Gestalt biologischer Zellen 2, in dem der Abstand zwischen der Haltereinrichtung 11 und der Wanne 23 verringert wird, bis der hängende Tropfen 3 in die Polymerisationssubstanz 5 eintaucht. Die Erfinder haben festgestellt, dass beim Eintauchen instantan die Polymerisierung an der Oberfläche des hängenden Tropfen beginnt, so dass dessen Gestalt auch beim Eintauchen in die Polymerisationssubstanz 5 erhalten bleibt.

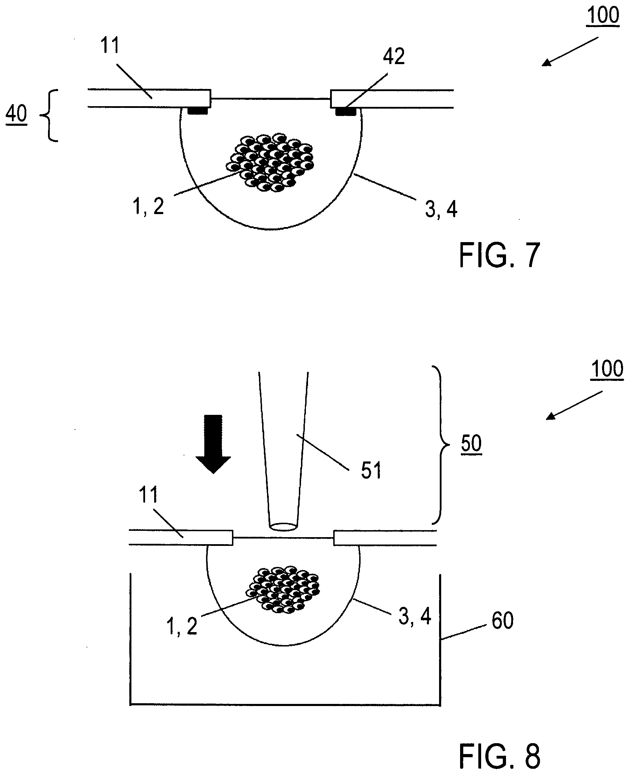

Durch die Zufuhr der Polymerisationssubstanz zu dem hängenden Tropfen 3 wird das gelöste Alginat vernetzt, so dass eine Polymerkapsel 4 gebildet wird (Figur 2). Nach der Polymerisierung bleibt die Polymerkapsel 4 an der Halteeinrichtung 11 haften. Für die weitere Verwendung der Polymerkapsel 4 wird diese von der Halteeinrichtung 11 mechanisch und/oder chemisch abgetrennt (siehe unten Figuren 8 bis 10).

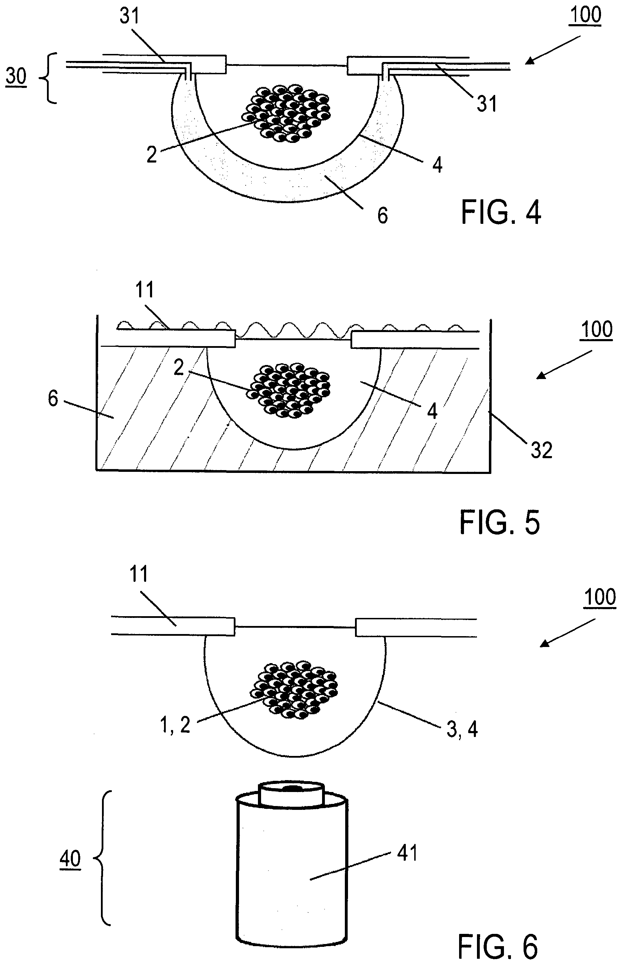

Figur 4 zeigt eine Ausführungsform der Erfindung, bei der die Verkapselungseinrichtung 100 mit einer Hülleinrichtung 30, umfassend mindestens eine Fluidleitung 31 zur Zufuhr einer Hüllsubstanz 6 zur Oberfläche der Polymerkapsel 4 ausgestattet ist. Beim dargestellten Beispiel sind zwei Fluidleitungen 31 fest mit der Halteeinrichtung 11 verbunden und so angeordnet, dass die Fluidleitungen 31 an die Oberfläche der Polymerkapsel 4 angrenzend mündet. Bei abgewandelten Varianten der Erfindung kann die Hülleinrichtung von der Halteeinrichtung getrennt angeordnet sein und zum Beispiel eine dritte Pipettiereinrichtung umfassen.

Die Umhüllung der Polymerkapsel 4 mit einer Hüllsubstanz kann verschiedene Funktionen haben. Beispielsweise kann die Oberfläche der Polymerkapsel 4 funktionalisiert, zum Beispiel zusätzlich gehärtet oder mit zusätzlichen chemischen Substanzen ausgestattet werden, oder es kann eine zusätzliche Polymerschicht aufgetragen werden. Die Hüllsubstanz wird vorzugsweise im flüssigen Zustand zugeführt und auf der Oberfläche der Polymerkapsel 4 verfestigt. Alternativ kann die Hüllsubstanz auf der Oberfläche der Polymerkapsel 4 im flüssigen Zustand bleiben.

Bei der Verkapselung biologischer Zellen 2 kann die Kultivierung der Zellen in der Polymerkapsel 4 fortgesetzt werden. In diesem Fall umfasst die Hüllsubstanz 6 vorzugsweise ein Kultivierungsmedium (Nähr- und/oder Differenzierungsmedium), welches durch das vernetzte Alginat der Polymerkapsel 4 zu den Zellen 2 diffundiert.

Gemäß einer weiteren Variante der Erfindung kann die Hülleinrichtung eine Wanne 32 zur Aufnahme der Hüllsubstanz 6 umfassen, wie schematisch in Figur 5 gezeigt ist. Die Hüllsubstanz 6 in der Wanne 32 umfasst zum Beispiel ein Kultivierungsmedium, das durch das vernetzte Alginat der Polymerkapsel 4 zu den Zellen 2 diffundiert. Vorzugsweise wird die gesamte Halteeinrichtung 11 in die Wanne 32 eingetaucht, so dass eine allseitige Benetzung der Polymerkapsel 4 mit dem Kultivierungsmedium erfolgt.

Wenn die Halteeinrichtung 11 eine Lochplatte mit einer Vielzahl von Löchern, zum Beispiel eine Tropfenplatte für die Kultivierung in hängenden Tropfen, umfasst, können alle Polymerkapseln 4 gleichzeitig in ein einheitliches Kultivierungsmedium oder selektiv jeweils in verschiedene Kultivierungsmedien eingetaucht werden. Beispielsweise können eine Vielzahl von Wannen 32 mit einer geometrischen Anordnung entsprechend der geometrischen Anordnung der Löcher in der Lochplatte der Halteeinrichtung 11 vorgesehen sein, wobei in den Wannen 32 als Hüllsubstanz 6 gleiche oder verschiedene Kultivierungsmedien angeordnet sind. Vorteilhafterweise kann diese Variante der Erfindung einfach mit kommerziell verfügbaren Komponenten, wie zum Beispiel einer Tropfenplatte für die Kultivierung von Zellen in hängenden Tropfen und einer passenden Mikrotiterplatte zur Bereitstellung der Wannen, zum Beispiel im "96-Well-Format" realisiert werden. In diesem Fall bildet jede Polymerkapsel einen Alginat-Bioreaktor mit einer anderen Medienzusammensetzung, was insbesondere für Screening-Anwendungen von Vorteil ist.

Figur 6 zeigt schematisch, dass gemäß einer weiteren Ausführungsform der Erfindung die Verkapselungseinrichtung 100 mit einer Messeinrichtung 40 ausgestattet sein kann. Die Messeinrichtung 40 umfasst zum Beispiel ein Mikroskop 41 (teilweise dargestellt) zur optischen Erfassung des Zustands der Probe 1 im hängenden Tropfen 3 beziehungsweise der Polymerkapsel 4. Mit dem Mikroskop 41 kann insbesondere die weitere Kultivierung von Zellen 2 in der Polymerkapsel 4 überwacht werden.

Gemäß einer alternativen Variante der Erfindung, die in Figur 7 gezeigt ist, kann die Messeinrichtung 40 für eine elektrische Messung an den hängenden Tropfen 3 beziehungsweise der Polymerkapsel 4 ausgelegt sein. In diesem Fall ist die Halteeinrichtung 11 mit Elektroden 42 ausgestattet, die an das Loch in der Lochplatte angrenzend angeordnet und mit einer Impedanzmesseinrichtung (nicht dargestellt) verbunden sind. Vorteilhafterweise kann mit einer Impedanzmessung der Zustand der Probe 1 im hängenden Tropfen 3, zum Beispiel die Anzahl der Zellen 2, detektiert werden.

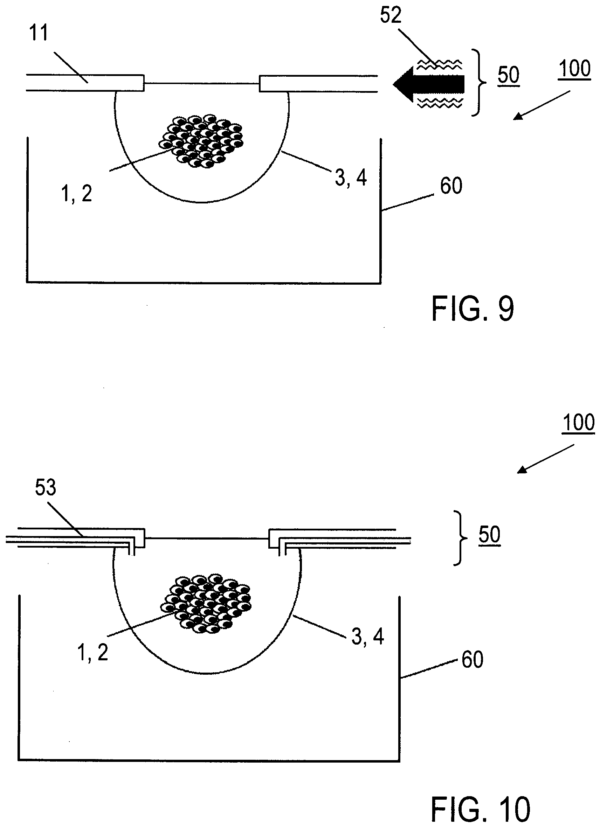

Die Figuren 8 bis 10 zeigen verschiedene Varianten der Abtrennung der Polymerkapseln 4 von der Halteeinrichtung 11 mit mechanischen und/oder chemischen Mitteln. Für die Abtrennung ist die Verkapselungseinrichtung 100 vorzugsweise mit einer Trenneinrichtung 50 ausgestattet, die zum Beispiel eine weitere Pipettiereinrichtung 51 (Figur 8), einen Ultraschallgenerator 52 (Figur 9) oder Fluidleitungen 53, die mit der Halteeinrichtung 11 verbunden sind (Figur 10), umfasst. Gemäß Figur 8 wird die Polymerkapsel 4 durch eine mechanische Kraft, die mit der weiteren Pipettiereinrichtung 51 ausgeübt wird, in ein Sammelgefäß 60 bewegt. Mit dem Ultraschallgenerator 52 kann die Halteeinrichtung 11 in Schwingungen versetzt werden, um die Polymerkapsel 4 abzuschütteln, so dass sie in das Sammelgefäß 60 fällt. Schließlich kann über die Fluidleitungen 53 eine depolymerisierende Flüssigkeit, wie z. B. EDTA zugeführt werden, unter deren Wirkung die Polymerkapsel 4 in den an die Halteeinrichtung 11 angrenzenden Abschnitten aufgelöst wird, so dass sie ebenfalls in das Sammelgefäß 60 fällt.

Die Anwendung der erfindungsgemäßen Verkapselung kann zum Beispiel in Verbindung mit dem folgenden Prozess zur Kultivierung biologischer Zellen erfolgen. In einem konkreten Beispiel ist zunächst eine Differenzierung von hESCs oder hiPSCs-Stammzellen in Kardiomyozyten mit einer anschließenden Durchführung eines Medikamenten-Zytotoxizitätstests (so genannter "embryonic stem cell test, EST") vorgesehen.

Tropfen (20 - 40 µl) einer Zellsuspension aus hESCs/hiPSCs werden mit Hilfe eines Pipettierroboters an die Tropfenplatte der Halteeinrichtung 11 gesetzt. Innerhalb von drei Tagen bilden sich so genannte embryoide Körper (EBs, Sphäroide) aus den Zellen. Anschließend können zu diesen multizellulären Aggregaten optional Microcarrier mit Hilfe des Pipettierroboters zugefügt werden, so dass es zu einer Adhäsion der EBs auf den Oberflächen der Microcarrier kommt.

Im nächsten Prozessschritt wird das Medium der EBs bzw. der auf Microcarriern adhärierten EBs durch eine Alginatlösung getauscht und mit dem erfindungsgemäßen Verfahren vernetzt. Die an der Tropfenplatte befestigten, verkapselten EBs werden nun erneut mit Hilfe von Mikrokanälen oder durch das Eintauchen der kompletten Tropfenplatte mit Differenzierungsmedium versorgt.

Nach der erfolgreichen Differenzierung der Zellen in Kardiomyozyten, erkennbar durch ein rhytmisches Schlagen und Zucken der Zellaggregate, kann ein Screening von Wirkstoffen nach zytotoxischen Effekten stattfinden. Hierbei können die Wirkstoffe erneut durch Mikrokanäle oder durch das Eintauchen der Platte in eine Multiwellplatte mit getrennten Kavitäten, so dass jeder Tropfen separat einer Wirkstoffkonzentration bzw. -zusammensetzung ausgesetzt wird, zugeführt werden.

Weitere Beispiele für eine Anwendung der erfindungsgemäßen Verkapselung sind eine Optimierung der Differenzierungseffizienz von MSCs in Osteoblasten, eine Kultivierung von funktionalen Chondrozyten, oder eine Produktion von verkapselten Zellen aus hESCs/hiPSCs für eine immunisolierte Transplantation.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Verkapselungseinrichtung (100), die zur Verkapselung einer Probe (1, 2) in einer Polymerkapsel (4) eingerichtet ist, umfassend:
- einen Tropfengenerator (10), der für eine Bereitstellung eines Tropfens (3) einer Suspension eingerichtet ist, der die Probe (1) enthält, und
- eine Vernetzungseinrichtung (20), die zur Polymerisierung des Tropfens (3) eingerichtet ist,
**dadurch gekennzeichnet, dass**
- der Tropfengenerator (10) eine Halteeinrichtung (11) aufweist, die zur Aufnahme des Tropfens (3) in einem hängenden Zustand eingerichtet ist, und
- die Vernetzungseinrichtung (20) angeordnet ist, eine Polymerisationssubstanz (5) zu dem hängenden Tropfen (3) an der Halteeinrichtung (11) zuzuführen und die Polymerkapsel (4) zu bilden.

2. Verkapselungseinrichtung gemäß Anspruch 1, bei der die Halteeinrichtung (11) mindestens eines umfasst von
- einer Halteplatte mit einer freiliegenden Unterseite, die für die Aufnahme des hängenden Tropfens (3) eingerichtet ist,
- einer Lochplatte, die ein Loch für die Aufnahme des hängenden Tropfens (3) aufweist,
- einer Netzplatte, die für die Aufnahme des hängenden Tropfens (3) eingerichtet ist, und
- einem Haltestab, der für die Aufnahme des hängenden Tropfens (3) eingerichtet ist.

3. Verkapselungseinrichtung gemäß einem der vorhergehenden Ansprüche, bei der
- der Tropfengenerator (10) eine Zufuhreinrichtung aufweist, die zur Zufuhr der Suspension zu der Halteeinrichtung (11) und zur Bildung des hängenden Tropfens (3) an der Halteeinrichtung (11) eingerichtet ist.

4. Verkapselungseinrichtung gemäß Anspruch 3, bei der
- die Zufuhreinrichtung (12) eine erste Pipettiereinrichtung umfasst, die für ein Setzen des hängenden Tropfens (3) an der Halteeinrichtung (11) eingerichtet ist.

5. Verkapselungseinrichtung gemäß einem der vorhergehenden Ansprüche, bei der die Vernetzungseinrichtung (20) mindestens eines umfasst von
- einer Partikelschusseinrichtung (21), die für ein Einschießen der Polymerisationssubstanz (5) in den hängenden Tropfen (3) eingerichtet ist,
- einer zweiten Pipettiereinrichtung (22), die für eine Einleitung der Polymerisationssubstanz (5) in den hängenden Tropfen (3) eingerichtet ist, und
- einer Wanne (23) zur Aufnahme der Polymerisationssubstanz (5), wobei die Halteeinrichtung (11) und die Wanne derart relativ zueinander beweglich angeordnet sind, dass der hängende Tropfen (3) in die Polymerisationssubstanz (5) in der Wanne eingetaucht werden kann.

6. Verkapselungseinrichtung gemäß einem der vorhergehenden Ansprüche, bei der
- eine Hülleinrichtung (30) vorgesehen ist, die zur Umhüllung der Polymerkapsel (4) mit einer Hüllsubstanz (6) eingerichtet ist.

7. Verkapselungseinrichtung gemäß Anspruch 6, bei der
- die Hülleinrichtung (30) mindestens eine Fluidleitung (31) umfasst, die mit der Halteeinrichtung (11) fest verbunden ist.

8. Verkapselungseinrichtung gemäß einem der vorhergehenden Ansprüche, die umfasst
- eine Messeinrichtung (40), die für eine Untersuchung des hängenden Tropfens (3) und/oder der Polymerkapsel (4) eingerichtet ist, und/oder
- eine Trenneinrichtung (50), die zur Abtrennung der Polymerkapsel (4) von der Haltereinrichtung eingerichtet ist.

9. Verfahren zur Verkapselung einer Probe (1, 2) in einer Polymerkapsel (4), umfassend die Schritte:
- Bereitstellung eines Tropfens (3) einer Suspension, der die Probe (1, 2) enthält, und
- Polymerisierung des Tropfens (3),
**dadurch gekennzeichnet, dass**
- der Tropfen (3) in einem hängenden Zustand bereitgestellt werden, und
- die Polymerisierung eine Zufuhr einer Polymerisationssubstanz (5) zu dem hängenden Tropfen (3) und eine Polymerisationsreaktion in dem Tropfen (3) umfasst, so dass die Polymerkapsel (4) gebildet wird.

10. Verfahren gemäß Anspruch 9, bei dem die Bereitstellung des hängenden Tropfens (3) mindestens eines umfasst von
- Aufnahme des hängenden Tropfens (3) an einer freiliegenden Unterseite einer Halteplatte,
- Aufnahme des hängenden Tropfens (3) in den Löcher einer Lochplatte,
- Aufnahme des hängenden Tropfens (3) an einer freiliegenden Unterseite einer Netzplatte, und
- Aufnahme des hängenden Tropfens (3) an einem Haltestab.

11. Verfahren gemäß einem der Ansprüche 9 bis 10, bei dem
- die Bereitstellung des hängenden Tropfens (3) ein Setzen des hängenden Tropfens (3) mit einer ersten Pipettiereinrichtung an der Halteeinrichtung (11) umfasst.

12. Verfahren gemäß einem der Ansprüche 9 bis 11, bei dem die Polymerisierung mindestens eines umfasst von
- Einleitung der Polymerisationssubstanz (5) in den hängenden Tropfen (3) mit einer zweiten Pipettiereinrichtung (22),
- Einschießen der Polymerisationssubstanz (5) in den hängenden Tropfen (3) mit einer Partikelschusseinrichtung (21), und
- Eintauchen des hängenden Tropfens (3) in die Polymerisationssubstanz (5) in einer Wanne (23).

13. Verfahren gemäß einem der Ansprüche 9 bis 12, mit mindestens einem der Schritte
- Umhüllung der Polymerkapsel (4) mit einer Hüllsubstanz (6), und
- Untersuchung des hängenden Tropfens (3) oder der Polymerkapsel (4),
- Abtrennung der Polymerkapsel (4) von der Halteeinrichtung (11).

14. Verfahren gemäß einem der Ansprüche 9 bis 13, bei dem
- die Suspension eine Alginatlösung und die Polymerisationssubstanz (5) vernetzende Ionen enthalten, mit denen die Alginatlösung im hängenden Tropfen (3) polymerisiert wird, oder
- die Suspension vernetzende Ionen enthält und die Polymerisationssubstanz (5) eine Alginatlösung umfasst, die im hängenden Tropfen (3) polymerisiert wird.

15. Verfahren gemäß einem der Ansprüche 9 bis 14, bei dem
- die Probe mindestens eine biologische Zelle (2), mindestens ein Zellbestandteil und/oder mindestens eine Zellgruppe enthält.

## Claims

1. Encapsulation device (100), which is adapted to encapsulate a sample (1, 2) in a polymer capsule (4), comprising:
- a drop generator (10), which is adapted to provide a drop (3) of a suspension, wherein the drop (3) contains the sample (1), and
- a cross-linking device (20), which is adapted to polymerize the drop (3),
**characterized in that**
- the drop generator (10) has a holding device (11), which is adapted to receive the drop (3) in a hanging state, and
- the cross-linking device (20) is arranged to supply a polymerization substance (5) to the hanging drop (3) on the holding device (11) and to form the polymer capsule (4).

2. Encapsulation device according to claim 1, in which the holding device (11) comprises at least one of
- a holding plate having an exposed underside, which is adapted to receive the hanging drop (3),
- a hole plate, which has a hole for receiving the hanging drop (3),
- a mesh plate, which is adapted to receive the hanging drop (3), and
- a holding rod, which is adapted to receive the hanging drop (3).

3. Encapsulation device according to one of the preceding claims, in which
- the drop generator (10) has a feed device, which is adapted to feed the suspension to the holding device (11) and to form the hanging drop (3) on the holding device (11).

4. Encapsulation device according to claim 3, in which
- the feed device (12) comprises a first pipetting device, which is adapted to place the hanging drop (3) on the holding device (11).

5. Encapsulation device according to one of the preceding claims, in which the cross-linking device (20) comprises at least one of
- a particle shooting device (21), which is adapted to inject the polymerization substance (5) into the hanging drop (3),
- a second pipetting device (22), which is adapted to introduce the polymerization substance (5) into the hanging drop (3), and
- a bowl (23) for receiving the polymerization substance (5), the holding device (11) and the bowl being disposed so as to be movable relative to each other, such that the hanging drop (3) can be immersed in the polymerization substance (5) in the bowl.

6. Encapsulation device according to one of the preceding claims, in which
- a covering device (30) is provided, which is adapted to cover the polymer capsule (4) with a covering substance (6).

7. Encapsulation device according to claim 6, in which
- the covering device (30) comprises at least one fluid line (31), which is fixedly connected to the holding device (11).

8. Encapsulation device according to one of the preceding claims, comprising
- a measuring device (40), which is adapted to examine the hanging drop (3) and/or the polymer capsule (4), and/or
- a separating device (50), which is adapted to part-off the polymer capsule (4) from the holding device.

9. Method for encapsulating a sample (1, 2) in a polymer capsule (4), comprising the steps:
- providing a drop (3) of a suspension, wherein the drop (3) contains the sample (1, 2), and
- polymerizing the drop (3),
**characterized in that**
- the drop (3) is provided in a hanging state, and
- the polymerizing comprises a supply of a polymerization substance (5) to the hanging drop (3) and a polymerization reaction in the drop (3), such that the polymer capsule (4) is formed.

10. Method according to claim 9, in which the providing of the hanging drop (3) comprises at least one of
- receiving the hanging drop (3) on an exposed underside of a holding plate,
- receiving the hanging drop (3) in the holes of a hole plate,
- receiving the hanging drop (3) on an exposed underside of a mesh plate, and
- receiving the hanging drop (3) on a holding rod.

11. Method according to one of the claims 9 to 10, in which
- the providing of the hanging drop (3) comprises placing the hanging drop (3) on the holding device (11) by means of a first pipetting device.

12. Method according to one of the claims 9 to 11, in which the polymerizing comprises at least one of
- introducing the polymerization substance (5) into the hanging drop (3) by means of a second pipetting device (22),
- injecting the polymerization substance (5) into the hanging drop (3) by means of a particle shooting device (21), and
- immersing the hanging drop (3) in the polymerization substance (5) in a bowl (23).

13. Method according to one of claims 9 to 12, comprising at least one of the steps
- covering the polymer capsule (4) with a covering substance (6), and
- examining the hanging drop (3) or the polymer capsule (4),
- separating the polymer capsule (4) from the holding device (11).

14. Method according to one of claims 9 to 13, in which
- the suspension contains an alginate solution, and the polymerization substance (5) contains cross-linking ions by means of which the alginate solution is polymerized in the hanging drop (3), or
- the suspension contains cross-linking ions, and the polymerization substance (5) contains an alginate solution, which is polymerized in the hanging drop (3).

15. Method according to one of claims 9 to 14, in which
- the sample contains at least one biological cell (2), at least one cell component and/or at least one cell group.

## Revendications

1. Dispositif d'encapsulation (100), qui est mis au point pour encapsuler un échantillon (1, 2) dans une capsule de polymère (4), comprenant :
- un générateur de goutte (10), qui est mis au point pour fournir une goutte (3) d'une suspension qui contient l'échantillon (1), et
- un dispositif de réticulation (20), qui est mis au point pour polymériser la goutte (3),
**caractérisé en ce que**
- le générateur de goutte (10) présente un dispositif de retenue (11), qui est mis au point pour recevoir la goutte (3) dans un état suspendu, et
- le dispositif de réticulation (20) est disposé pour amener une substance de polymérisation (5) à la goutte (3) en suspension au niveau du dispositif de retenue (11) et pour former la capsule de polymère (4).

2. Dispositif d'encapsulation selon la revendication 1, dans lequel le dispositif de retenue (11) comprend au moins un élément parmi
- une plaque de retenue pourvue d'un côté inférieur dégagé, qui est mis au point pour recevoir la goutte (3) en suspension,
- une plaque perforée, qui présente un trou pour recevoir la goutte (3) en suspension,
- une plaque en filet, qui est mise au point pour recevoir la goutte (3) en suspension, et
- une barre de retenue, qui est mise au point pour recevoir la goutte (3) en suspension.

3. Dispositif d'encapsulation selon l'une quelconque des revendications précédentes, dans lequel
- le générateur de goutte (10) présente un dispositif d'amenée, qui est mis au point pour amener la suspension au dispositif de retenue (11) et pour former la goutte (3) en suspension au niveau du dispositif de retenue (11).

4. Dispositif d'encapsulation selon la revendication 3, dans lequel
- le dispositif d'amenée (12) comprend un premier dispositif de pipetage, qui est mis au point pour poser la goutte (3) en suspension au niveau du dispositif de retenue (11).

5. Dispositif d'encapsulation selon l'une quelconque des revendications précédentes, dans lequel le dispositif d'encapsulation (20) comprend au moins un élément parmi
- un dispositif de pulvérisation de particules (21), qui est mis au point pour injecter la substance de polymérisation (5) dans la goutte (3) en suspension,
- un deuxième dispositif de pipetage (22), qui est mis au point pour introduire la substance de polymérisation (5) dans la goutte (3) en suspension, et
- une cuve (23) servant à recevoir la substance de polymérisation (5), dans lequel le dispositif de retenue (11) et la cuve sont disposés de manière mobile l'un par rapport à l'autre de telle manière que la goutte (3) en suspension peut être plongée dans la substance de polymérisation (5) dans la cuve.

6. Dispositif d'encapsulation selon l'une quelconque des revendications précédentes, dans lequel
- est prévu un dispositif d'enveloppement (30) qui est mis au point pour envelopper la capsule de polymère (4) d'une substance d'enveloppement (6).

7. Dispositif d'encapsulation selon la revendication 6, dans lequel
- le dispositif d'enveloppement (30) comprend au moins un conduit de fluide (31), qui est relié de manière ferme au dispositif de retenue (11).

8. Dispositif d'encapsulation selon l'une quelconque des revendications précédentes, qui comprend :
- un dispositif de mesure (40), qui est mis au point pour examiner la goutte (3) en suspension et/ou la capsule de polymère (4), et/ou
- un dispositif de séparation (50), qui est mis au point pour retirer la capsule de polymère (4) du dispositif de retenue.

9. Procédé servant à l'encapsulation d'un échantillon (1, 2) dans une capsule de polymère (4), comprenant les étapes consistant à :
- fournir une goutte (3) d'une suspension, qui contient l'échantillon (1, 2), et
- polymériser la goutte (3),
**caractérisé en ce que**
- la goutte (3) est fournie dans un état de suspension, et
- la polymérisation comprend une amenée d'une substance de polymérisation (5) vers la goutte (3) en suspension et comprend une réaction de polymérisation dans la goutte (3) de sorte que la capsule de polymère (4) est formée.

10. Procédé selon la revendication 9, dans lequel la fourniture de la goutte (3) en suspension comprend au moins une étape parmi
- la réception de la goutte (3) en suspension au niveau d'un côté inférieur dégagé d'une plaque de retenue,
- la réception de la goutte (3) en suspension dans les trous d'une plaque perforée,
- la réception de la goutte (3) en suspension au niveau d'un côté inférieur dégagé d'une plaque de filet, et
- la réception de la goutte (3) en suspension au niveau d'une barre de retenue.

11. Procédé selon l'une quelconque des revendications 9 à 10, dans lequel
- la fourniture de la goutte (3) en suspension comprend une pose de la goutte (3) en suspension avec un premier dispositif de pipetage au niveau du dispositif de retenue (11).

12. Procédé selon l'une quelconque des revendications 9 à 11, dans lequel
la polymérisation comprend au moins une étape parmi
- l'introduction de la substance de polymérisation (5) dans la goutte (3) en suspension avec un deuxième dispositif de pipetage (22),
- le glissement de la substance de polymérisation (5) dans la goutte (3) en suspension avec un dispositif de pulvérisation de particules (21), et
- l'immersion de la goutte (3) en suspension dans la substance de polymérisation (5) dans une cuve (23).

13. Procédé selon l'une quelconque des revendications 9 à 12, comprenant au moins une des étapes suivantes
- l'enveloppement de la capsule de polymère (4) avec une substance d'enveloppement (6), et
- l'examen de la goutte (3) en suspension ou de la capsule de polymère (4),
- le retrait de la capsule de polymère (4) du dispositif de retenue (11).

14. Procédé selon l'une quelconque des revendications 9 à 13, dans lequel
- la suspension contient une solution d'alginate et la substance de polymérisation (5) contient des ions de réticulation, avec lesquels la solution d'alginate est polymérisée dans la goutte (3) en suspension, ou
- la suspension contient des ions de réticulation et la substance de polymérisation (5) comprend une solution d'alginate, qui est polymérisée dans la goutte (3) en suspension.

15. Procédé selon l'une quelconque des revendications 9 à 14, dans lequel
- l'échantillon contient au moins une cellule (2) biologique, au moins un constituant cellulaire et/ou au moins un groupe de cellules.
